# EUROPÄISCHE PATENTSCHRIFT

(11) **EP 1 778 828 B1**
(45) Veröffentlichungstag und Bekanntmachung des Hinweises auf die Patenterteilung: **19.12.2012**
(21) Anmeldenummer: 05767667.8
(22) Anmeldetag: 10.08.2005
(51) Int. Cl.: C12M 1/00, C12M 1/34

(54) **BIOREAKTOR**
BIOREACTOR
BIOREACTEUR

(30) Priorität: 16.08.2004 CH 135204
(43) Veröffentlichungstag der Anmeldung: 02.05.2007
(73) Patentinhaber: Sartorius Stedim Switzerland AG, 8317 Tagelswangen (CH)
(72) Erfinder: Röll, Marcel, CH-8124 Maur ZH (CH)
(74) Vertreter: Schmauder, Klaus Dieter
(86) Internationale Anmeldenummer: PCT/CH2005/000464
(87) Internationale Veröffentlichungsnummer: WO 2006/017951

(56) Entgegenhaltungen:
- EP-A- 0 810 281
- WO-A-00/66706
- WO-A-93/15402
- US-A- 5 164 796
- US-B1- 6 730 471

## Beschreibung

### Technisches Gebiet

Die Erfindung betrifft einen Bioreaktor gemäss Oberbegriff des Anspruches 1.

### Stand der Technik '

Ein Bioreaktor der eingangs genannten Art ist beispielsweise aus der US 6 730 471 B bekannt. Der Reaktorbehälter enthält an seinem Boden einen in das Innere des Reaktorbehälters weisenden angeformten Stutzen, der eine Sensormembrane trägt, die mit dem Reaktionsgut in Verbindung steht. An den Stutzen ist eine Fiberoptik anschliessbar, die mit einem Anzeiggerät verbunden ist. Ein solcher Anschlussstuzten ist für einen beutelartigen Reaktor nicht geeignet, da am Beutel ein Anschlussstutzen wegen der Flexibilität der Wandung nicht anformbar ist. Überdies muss der beutelartige Reaktor sich auf einer Unterlage abstützen, sodass die Unterseite für die Anordnung des Anschlussstutzens ebenfalls nicht geeignet ist. Die Druckschrift liefert keine Anhaltspunkte für die Anordnung eines elektrooptischen Sensors an einem als Beutel ausgebildeten Reaktor.

Ein als Beutel ausgebildeter Reaktorbehälter ist beispielsweise aus der WO 00/66706 bekannt. Dieser beutelartige Reaktorbehälter weist Anschlüsse auf, von denen einer insbesondere auch zur Einführung eines Sensors zur Ermittlung von Eigenschaften des Reaktiorisgutes geeignet ist. Nachteilig ist es, dass einerseits der Anschluss und andererseits der Sensor, der durch den Anschluss in das Behälterinnere eingebracht wird, steril gehalten werden müssen. Diese Ausbildung beinhaltet stets die Gefahr, dass durch den Sensor Verunreinigungen in den Reaktorbehälter und damit in das Reaktionsgut gelangen.

### Darstellung der Erfindung

Aufgabe der Erfindung ist es, einen Bioreaktor der eingangs genannten Art zu verbessern, um ihn als Beutel auszubilden.

Gelöst wird die Aufgabe durch die kennzeichnenden Merkmale des Anspruches 1.

Dadurch, dass der Aufnahmestutzen an der Oberseite des Reaktorbeutels angeordnet ist, ist die für einen Beutel notwendige untere Auflagefläche nicht beeinträchtigt. Der mit der Behälterwandung dicht verbundene Flanschteil ermöglicht einen sicheren Anschluss an die flexible Wandung des Beutels. Der vorzugsweise schlauchartige Verbindungsteil stellt die Verbindung mit dem Sensorteil her. Die Länge des Verbindungsteiles ist derart, dass der Sensorteil von oben in das Reaktionsgut reicht.

Der das Indikatorplättchen enthaltende Aufnahmestutzen kann zusammen mit dem Inneren des Reaktorbehälters sterilisiert werden. Es ist nicht mehr erforderlich, dass die Fiberoptik einer besonderen Sterilisation ausgesetzt werden muss, da sie mit dem Behälterinneren und damit mit dem Reaktionsgut nicht in Berührung kommt.

Vorteilhafte Ausgestaltungen des Biöreaktors sind in den Ansprüchen 2 bis 9 beschrieben.

Es ist von besonderem Vorteil, wenn der Aufnahmestutzen gemäss Anspruch 2 im mittleren Bereich des Reaktorbeutels angeordnet ist, dadurch wird sicher gestellt, dass der Sensor auch bei den Schwingbewegungen, denen der Bioreaktor während des Reaktionsprozesses üblicherweise ausgesetzt wird, stets mit dem Reaktionsgut in Berührung bleibt.

Um die eingesetzte Fiberoptik am Aufnahmestutzen fest zu halten und bezüglich des Indikatorplättchens auszurichten, ist es vorteilhaft, wenn der Bioreaktor nach Anspruch 3 Mittel zum Arretieren der eingesetzten Fiberoptik aufweist.

Gemäss Anspruch 4 kann der lichtdurchlässige Sensorteil einen Endteil mit einem Boden aufweisen, wobei der Boden direkt durch das Indikatorplättchen gebildet sein kann. Es ist aber auch möglich, das Indikatorplättchen in einer Kappe anzuordnen, die den Endteil des Sensorteils umschliesst.

Gemäss Anspruch 5 kann der Reaktorbehälter parallel zum ersten Aufnahmestutzen einen analogen zweiten Aufnahmestutzen mit einem Indikatorplättchen für eine einsetzbare Fiberoptik zur Ermittlung einer weiteren Eigenschaft des Reaktionsgutes aufweisen.

Das Indikatorplättchen ist in bekannter Weise ausgebildet und ändert seine optischen Eigenschaften in Abhängigkeit von den zu erfassenden Eigenschaften und Konzentrationen des zu bestimmenden Wertes. Dementsprechend wird auch der von der Fiberoptik auf das Indikatorplättchen ausgesandte Lichtstrahl unterschiedlich reflektiert und an das Anzeigegerät zurück gesandt, worauf dieses die Änderungen der optischen Eigenschaften des Indikatorplättchens entsprechend auswertet. Das Indikatorplättchen kann gemäss Anspruch 6 beispielsweise zur Ermittlung des Sauerstoffgehaltes des Reaktionsgutes ausgebildet sein. Das Indikatorplättchen kann aber auch gemäss Anspruch 7 zur Ermittlung des ph-Wertes des Reaktionsgutes ausgestaltet sein.

Ein besonders bevorzugter Bioreaktor ist in Anspruch 8 beschrieben, wonach der Reaktorbeutel aus Kunststoff ausgebildet ist, der an zwei einander gegenüber liegenden Seiten in einen Träger einspannbar ist. Dabei ist die Ausgestaltung nach Anspruch 9 von Vorteil, wonach die einspannbaren Seiten jeweils einen Saum aufweisen, in dem eine quer zur Einspannrichtung verlaufende Verdickung vorhanden ist.

### Kurze Beschreibung der Zeichnungen

Ausführungsbeispiele der Erfindung werden nachfolgend anhand der Zeichnungen näher beschrieben, dabei zeigen:
- Figur 1: einen Bioreaktor im Vertikalschnitt;
- Figur 2: einen Anschlussstutzen des Bioreaktors im Vertikalschnitt und in grösserem Massstab;
- Figur 3: den Sensorteil des Anschlussstutzens gemäss Figur 2 im Schnitt III-III.

### Wege zur Ausführung der Erfindung

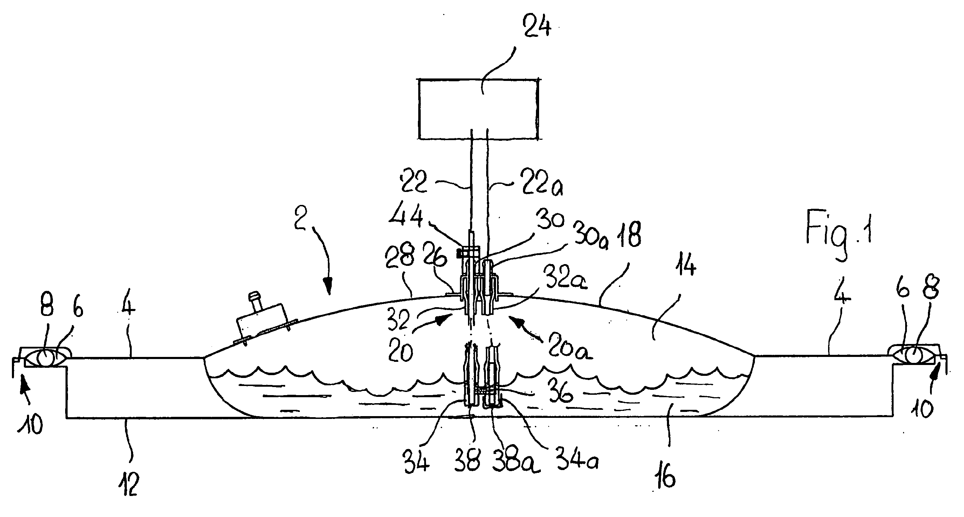

Die Figur 1 zeigt einen Bioreaktor mit einem Reaktorbeutel 2, der an zwei einander gegenüberliegenden Seiten 4 jeweils einen Saum 6 aufweist, in dem eine Verdickung 8 vorhanden ist. Der mit der Verdickung 8 ausgestattete Saum 6 ist in einer Einspannvorrichtung 10 eines Trägers 12 für den Reaktorbeutel eingespannt

Der Innenraum 14 des Reaktorbeutels 2 dient zur Aufnahme eines Reaktionsgutes 16, das beispielsweise ein biologisches Material sein kann. An der Oberseite 18 des Reaktorbeutels 2 ist ein Aufnahmestutzen 20, der hier in Zwillingsanordnung gezeigt ist, angeordnet und ragt in den Innenraum 14 des Reaktorbeutels bis in das Reaktionsgut 16. In die beiden Aufnahmestutzen 20,20a ist jeweils eine Fiberoptik 22,22a eingesetzt, die zu einem Anzeigegerät 24 führen, um die Reaktionssignale der Fiberoptik auszuwerten und bestimmte Eigenschaften des Reaktionsgutes zu ermitteln.

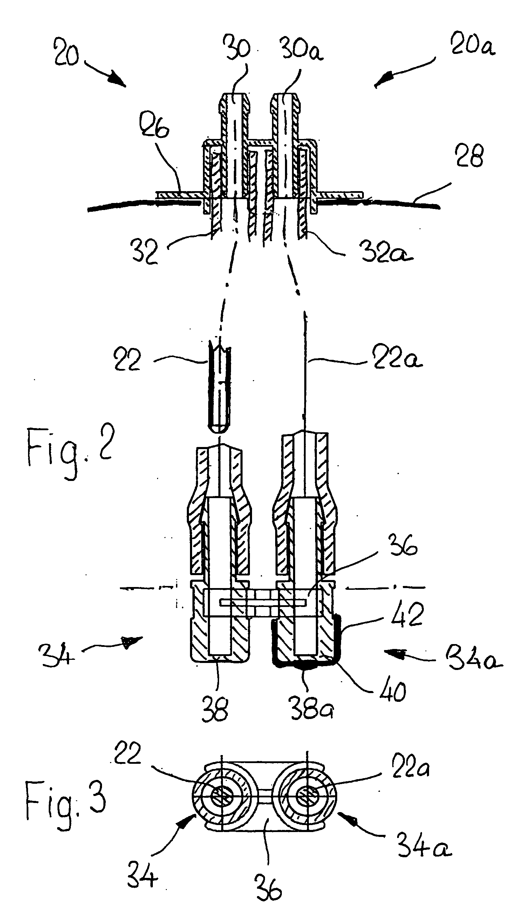

Die Aufnahmestutzen 20,20a sind in den Figuren 2 und 3 näher dargestellt. Sie enthalten einen Flanschteil 26, der mit der Wandung 28 des Reaktorbeutels 2 mediumsdicht verbunden ist. Der Flanschteil 26 enthält Rohrstutzen 30,30a, an denen im Innenraum 14 des Reaktorbeutels 2 schlauchartige Verbindungsteile 32,32a angeschlossen sind, die zu lichtdurchlässigen Sensorteilen 34,34a führen, mit denen sie wiederum mediumsdicht verbunden sind. Die Sensorteile 34,34a werden über eine Klammer 36 zusammen gehalten. Der Sensorteil 34 enthält am Boden ein eingegossenes oder angeklebtes Indikatorplättchen 38, welches mit dem Innenraum 14 des Reaktorbeutels 2 und damit auch mit dem Reaktionsgut 16 in Verbindung steht. Der Sensorteil 34a kann analog dem Sensorteil 34 ausgestaltet sein, im vorliegenden Beispiel ist das Indikatorplättchen 38a jedoch nicht im Boden 40 des Sensorteiles 34 angeordnet, sondern in einer Kappe 42, welche den Sensorteil umschliesst. Der Boden 40 ist lichtdurchlässig ausgestaltet, so dass die optischen Eigenschaften des Indikatorplättchens 38a durch den Boden 40 von der Fiberoptik 22,22a betrachtet werden können. Der Aufnahmestutzen 20,20a mit seinem Verbindungsteil 32,32a und dem Sensorteil 34,34a ist bezüglich des Innenraumes 14 des Reaktorbeutels 2 mediumsdicht ausgestaltet, so dass der im Inneren des Reaktionsbehälters liegende Teil des Aufnahmestutzens mit dem Reaktorbeutel bei der Herstellung oder vor Ort sterilisiert werden kann. Bezüglich der Fiberoptik 22,22a ist eine Sterilisation nicht mehr erforderlich, sie kann vor Ort im Bedarfsfall ohne grosse Manipulation in den Aufnahmestutzen 20,20a eingesetzt werden. Ein für die Fiberoptik 22 dargestelltes Arretierglied 44 sorgt für die Arretierung der Fiberoptik 22,22a im Aufnahmestutzen 20,20a und deren Ausrichtung bezüglich der Indikatorplättchen 38,38a. Vom Anzeigegerät 24 ausgesandte Lichtstrahlen brechen dann an dem Indikatorplättchen 38,38a, worauf die reflektierten Strahlen an das Anzeigegerät 24 zurück gesandt und ausgewertet werden. Je nach zur Anwendung gelangenden Indikatorplättchen 38,38a können unterschiedliche Werte des Reaktionsgutes, wie z.B. der Sauerstoffgehalt oder der pH-Wert ermittelt und in der jeweiligen Grösse festgestellt werden.

Reaktorbeutel existieren in verschiedenen Grössen, so dass der Abstand der oberen Wandung zum Boden des Reaktorbeutels entsprechend variieren kann. Die Länge des Verbindungsteiles zwischen dem Änschlussstutzen und dem Sensorteil kann stets so gewählt werden, dass der Sensorteil in das Reaktionsgut eintaucht.

### Bezugszeichenliste

- 2: Reaktorbeutel
- 4: Seite
- 6: Saum
- 8: Verdickung
- 10: Einspannvorrichtung
- 12: Träger
- 14: Innenraum
- 16: Reaktionsgut
- 18: Oberseite
- 20: Aufnahmestutzen
- 20a: Aufnahmestutzen
- 22: Fiberoptik
- 22a: Fiberoptik
- 24: Anzeigegerät
- 26: Flanschteil
- 28: Wandung
- 30: Rohrstutzen
- 30a: Rohrstutzen
- 32: schlauchartiger Verbindungsteil
- 32a: schlauchartiger Verbindungsteil
- 34: Sensorteil
- 34a: Sensorteil
- 36: Klammer
- 38: Indikatorplättchen
- 38a: Indikatorplättchen
- 40: Boden
- 42: Kappe
- 44: Arretierglied

## Patentansprüche

1. Bioreaktor mit einem Reaktorbehälter (2) mit einem in den Behälterinnenraum (14) ragenden, gegen den Behälterinnenraum (14) mediumsdichten Aufnahmestutzen (20,20a), der im Behälterinnenraum (14) einen lichtdurchlässigen Sensorteil (34,34a) mit einem mit dem Behälterinhalt in Verbindung stehenden Indikatorplättchen (38,38a) enthält, das mittels einer in den Aufnahmestutzen (20,20a) einsetzbaren Fiberoptik abtastbar ist, welche mit einem Anzeigegerät (24) in Verbindung steht, **dadurch gekennzeichnet, dass** der Reaktorbehälter als Beutel ausgebildet ist, wobei der Aufnahmestutzen (20,20a) an der Oberseite des Reaktorbeutel angeordnet ist und einen mit der Behälterwandung (28) dicht verbundenen Flanschteil (26) aufweist, der über einen vorzugsweise schlauchartigen Verbindungsteil (32,23a) mit dem Sensorteil (34,34a) verbunden ist, derart, dass der Sensorteil (34,34a) von oben in das Reaktionsgut reicht.

2. Bioreaktor nach Anspruch 1, **dadurch gekennzeichnet, dass** der Aufnahmestutzen (20,20a) im mittleren Bereich des Reaktorbeutels angeordnet ist.

3. Bioreaktor nach Anspruch 1 oder 2, **dadurch gekennzeichnet, dass** der Aufnahmestutzen (20,20a) Mittel (44) zum Arretieren der eingesetzten Fiberoptik (22,22a) aufweist.

4. Bioreaktor nach einem der Ansprüche 1 bis 3, **dadurch gekennzeichnet, dass** der Sensorteil (34,34a) einen Endteil mit einem Boden (40) aufweist, wobei der Boden (40) direkt durch das Indikatorplättchen (38) gebildet ist oder in einer Kappe (42) enthalten ist, die den Endteil umschliesst.

5. Bioreaktor nach einem der Ansprüche 1 bis 4, **dadurch gekennzeichnet, dass** er parallel zum ersten Aufnahmestutzen (20) einen analogen zweiten Aufnahmestutzen (20a) mit einem Indikatorplättchen (38a) für eine einsetzbare Fiberoptik (22a) zur Ermittlung einer weiteren Eigenschaft des Reaktionsgutes aufweist.

6. Bioreaktor nach einem der Ansprüche 1 bis 5, **dadurch gekennzeichnet, dass** das Indikatorplättchen (38) zur Ermittlung des Sauerstoffgehaltes des Reaktionsgutes (16) ausgebildet ist.

7. Bioreaktor nach einem der Ansprüche 1 bis 5, **dadurch gekennzeichnet, dass** das Indikatorplättchen (38a) zur Ermittlung des pH - Wertes des Reaktionsgutes (16) ausgebildet ist.

8. Bioreaktor nach einem der Ansprüche 1 bis 7, **dadurch gekennzeichnet, dass** der Reaktorbeutel an zwei einander gegenüberliegenden Seiten (4) in einen Träger (12) einspannbar ist.

9. Bioreaktor nach Anspruch 8, **dadurch gekennzeichnet, dass** die einspannbaren Seiten (4) jeweils einen Saum (6) aufweisen, in dem eine quer zur Einspannrichtung verlaufende Verdickung (8) vorhanden ist.

## Claims

1. A bioreactor, comprising a reactor vessel (2) with a receiving port (20, 20a) that extends into the vessel interior (14) in medium-tight fashion against the vessel interior (14), comprising a transparent sensor piece (34, 34a) with an indicator tile (38, 38a) arranged in the vessel interior (14) in contact with the vessel contents, which indicator tile may be scanned by a fiber optic that may be introduced into the receiving port (20, 20a) and is contacted to a display device (24), **characterized in that** the reactor vessel is embodied as a bag, wherein the receiving port (20, 20a) is arranged at the upper side of the reactor bag and is provided with a flange piece (26) tightly sealed to the vessel wall (28) and connected to the sensor piece (34, 34a) via a preferably tubular connector piece (32, 23a) in such a way that the sensor piece (34, 34a) reaches into the reactant from above.

2. The bioreactor according to claim, 1 **characterized in that** the receiving port (20, 20a) is arranged in a central region of the reactor bag.

3. The bioreactor according to claim 1 or 2, **characterized in that** the receiving port (20, 20a) comprises means (44) for securing the inserted fiber optic (22, 22a).

4. The bioreactor according to any one of claims 1 to 3, **characterized in that** the sensor piece (34, 34a) comprises an end portion with a bottom (40), wherein the bottom (40) is directly formed by the indicator tile (38) or is incorporated within a cap (42) that encloses the end portion.

5. The bioreactor according to any one of claims 1 to 4, **characterized in that** it comprises an analogous second receiving port (20a) that is parallel to the first receiving port (20) and comprises an indicator tile (38a) for an insertable fiber optic (22a) for determining a further property of the reactant.

6. The bioreactor according to any one of claims 1 to 5, **characterized in that** the indicator tile (38) is designed for determining the oxygen content of the reactant (16).

7. The bioreactor according to any one of claims 1 to 5, **characterized in that** the indicator tile (38a) is designed for determining the pH value of the reactant (16).

8. The bioreactor according to any one of claims 1 to 7, **characterized in that** the reactor bag can be clamped at two opposite sides (4) thereof to a support (12).

9. The bioreactor according to claim 8, **characterized in that** the sides (4) which can be clamped each comprise a seam (6) which is provided with a bulged rim (8) extending transversally to the clamping direction.

## Revendications

1. Bioréacteur comportant une chambre de réaction (2) ayant un ajutage de réception (20, 20a) étanche à un milieu, venant en saillie dans le volume (14) de la chambre et en direction du volume de la chambre (14) comportant une partie de capteur (34, 34a) transparente dans la chambre de réaction (14) et une plaquette indicatrice (38, 38a) en contact avec le contenu de la chambre, cette plaquette étant détectée par une fibre optique introduite dans l'ajutage de réception (20, 20a), et en liaison avec l'appareil d'affichage (24),
bioréacteur **caractérisé en ce que**
la chambre de réaction est en forme de poche et l'ajutage de réception (20, 20a) est installé sur le côté supérieur de la poche du réacteur et comporte une bride (26) reliée de manière étanche à la paroi (28) de la chambre, cette bride étant reliée à la partie de capteur (34, 34a) de préférence par une pièce de liaison (32, 23a) en forme de tube de façon que la partie de capteur (34, 34a) arrive par le dessus dans le produit de réaction.

2. Bioréacteur selon la revendication 1,
**caractérisé en ce que**
l'ajutage de réception (20, 20a) est placé dans la région centrale de la poche de réaction.

3. Bioréacteur selon la revendication 1 ou 2,
**caractérisé en ce que**
l'ajutage de réception (20, 20a) comporte des moyens (44) pour bloquer la fibre optique (22, 22a) installée.

4. Bioréacteur selon l'une des revendications 1 à 3,
**caractérisé en ce que**
la partie de capteur (34, 34a) comporte une pièce d'extrémité avec un fond (40) formé directement par la plaquette indicatrice (38) ou contenu dans un capuchon (42) entourant la pièce d'extrémité.

5. Bioréacteur selon l'une des revendications 1 à 4,
**caractérisé en ce que**
parallèlement au premier ajutage de réception (20), il comporte un second ajutage de réception (20a), analogue, ayant une plaquette indicatrice (38a) pour une fibre optique (22a) installée et servant à déterminer une autre propriété du produit de réaction.

6. Bioréacteur selon l'une des revendications 1 à 5,
**caractérisé en ce que**
la plaquette indicatrice (38) détermine la teneur en oxygène du produit de réaction (16).

7. Bioréacteur selon l'une des revendications 1 à 5,
**caractérisé en ce que**
la plaquette indicatrice (38a) détermine le pH du produit de réaction (16).

8. Bioréacteur selon l'une des revendications 1 à 7,
**caractérisé en ce que**
la poche de réaction est enserrée dans un support (12) par deux cotées opposés (4).

9. Bioréacteur selon la revendication 8,
**caractérisé en ce que**
les côtés destinés à être tendus (4) ont chacun un ourlet (6) muni d'un renforcement (8) dirigé transversalement à la direction dans laquelle s'exerce la tension.
